# EUROPEAN PATENT APPLICATION

(11) **EP 2 762 146 A1**
(43) Date of publication of application: **06.08.2014**
(21) Application number: 14153484.2
(22) Date of filing: 31.01.2014
(51) Int. Cl.: A61K 35/64

(54) **Composition for Earache**

(30) Priority: 31.01.2013 DK 201370055
(71) Applicant: Unigroup ApS, 2800 Lyngby (DK)
(72) Inventor: Licht, Flemming, 2720 Vanløse (DK)
(74) Representative: Poulsen, Niels Jakob

(57) **Abstract**

The present invention relates to a composition for the treatment of earache. In particular, it concerns a composition for treatment of earache caused by otitis, such as otitis externa or otitis media.

## Description

The present invention relates to a composition for the treatment of earache. In particular, it concerns a composition for treatment of earache caused by otitis, such as otitis externa or otitis media.

### Technical Background

The International patent application WO 2012/065651 A1 relates to compositions of plant extracts for treating skin diseases involving abnormal cell growth.

### Summary of the invention

Pain is one of the symptoms that may indicate ear disease. Earache is mainly due to problems in either the outer ear (from the earlobe to the eardrum) or the middle ear (behind the eardrum).

Otitis media is inflammation of the middle ear, or middle ear infection. It occurs in the area between the tympanic membrane and the inner ear, including a duct known as the eustachian tube. It is one of the two most common causes of earache - the other being otitis externa. External otitis may *inter alia* be caused by microbial infection. Otitis media is most commonly caused by infection with viral, bacterial, or fungal pathogens.

Otitis externa (also known as "External otitis" and "Swimmer's ear") is an inflammation of the outer ear and ear canal. Inflammation of the skin of the ear canal is the essence of this disorder. The inflammation can be secondary to dermatitis (eczema) only, with no microbial infection, or it can be caused by active bacterial or fungal infection. In either case, but more often with infection, the ear canal skin swells and may become painful or tender to touch.

When the middle ear becomes acutely infected, pressure builds up behind the eardrum (tympanic membrane), frequently causing intense pain. It may result in bullous myringitis, which means that the tympanic membrane is blistered and inflamed. In severe or untreated cases, the tympanic membrane may rupture, allowing the pus in the middle ear space to drain into the ear canal. If there is enough of it, this drainage may be obvious.

Surprisingly, it has been discovered that a composition of the present invention may relieve earache, in particular caused by otitis, more particularly otitis externa or otitis media. Even more surprising, pain caused by otitis, such as otitis externa or otitis media, may be alleviated by application of a composition according to the invention in the outer part of the ear. Without being bound by any theory, it is speculated that honey and/or glycerol present in the present composition allows affecting the osmotic pressure over the eardrum, and thus relieve pressure. Further, honey may help reduce inflammation and reduce exudate formation more promptly than standard treatments. It is further speculated that flavonoids such as procyanidins may have a beneficial effect on inflammatory and/or viral conditions.

According to an aspect, the present application concerns a composition for the treatment of earache, said composition comprising a pharmaceutically acceptable hyperosmotic ingredient. According to another aspect, the present application concerns a composition comprising honey and optionally at least one additional ingredient for the treatment of earache. The composition is preferably a pharmaceutical composition.

According to yet another aspect, the present application concerns a composition comprising glycerol and optionally one or more additional ingredients for the treatment of earache.

According to an aspect, the present application concerns a composition comprising at least one tannin, preferably a flavonoid, and optionally at least one additional ingredient, for the treatment of earache.

According to an aspect, the present application concerns a method for the treatment of earache, comprising topical application of a composition according to the invention.

According to an aspect, the present application concerns the use of a hyperosmotic ingredient for the treatment of earache.

According to an aspect, the present application concerns the use of honey and/or glycerol for the treatment of earache.

### Detailed Disclosure

An ingredient or composition having an osmotic pressure higher than the osmotic pressure of a 0.9% sodium chloride solution is denoted "hyperosmotic". The term "hyperosmotic" is used here not only about solutions having a concentration of solute providing an osmotic pressure higher than the osmotic pressure of a 0.9% sodium chloride solution, but also about other compositions and ingredients. If the osmotic pressure is lower, it is denoted "hypoosmotic".

According to an embodiment, the present invention concerns a composition for the treatment of earache, said composition comprising a pharmaceutically acceptable hyperosmotic ingredient.

The composition is preferably a pharmaceutical composition.

Hyperosmotic ingredients comprise, but are not limited to: Salts, such as sodium chloride, sodium phosphate (and variants), potassium sodium tartrate, magnesium citrate, magnesium hydroxide (Milk of magnesia or Cream of magnesia), magnesium sulfate (Epsom salt); further glycerin, sorbitol, lactulose, polyethylene glycol (PEG), and solutions of polyethylene glycol and electrolytes (sodium chloride, sodium bicarbonate, potassium chloride, and sodium sulfate); Sugars or saccharides, examples include, but are not limited to, fructose, glucose, sucrose; mixtures or compositions, such as honey; and other ingredients, such as glycerol, etc.

According to another embodiment, the present invention concerns a composition, wherein said hyperosmotic ingredient is selected among honey and glycerol.

According to an embodiment, the present invention concerns a composition comprising honey and optionally at least one additional ingredient for the treatment of earache.

Honey is primarily fructose and glucose in that order, with a little sucrose (about 1%), and less than 10% other sugars, and about 17% water.

Honey is one of the earliest sweeteners used by humans. Beekeeping to obtain honey probably goes back to the early Egyptians who used honey in embalming, in medicine, and for food. Honey has long been a staple of the kitchen.

It is speculated that honey may act as an osmotic ingredient, relieving the eardrum of pressure. The antibacterial mechanisms of honey comprise the osmotic effect, slow-release of hydrogen peroxide, low acidity, presence of antioxidants, and presence of methylglyoxal (MGO) and bee defensin-1. It is speculated that one or more of these mechanisms may further confer anti-fungal properties to honey.

The honey employed should be of a quality needed for a pharmaceutical composition, free of contaminants such as the bacterium Clostridium botulinum.

The at least one ingredient may be any suitable excipient or active ingredient. The ingredient may be a solvent, and may have impact on the overall properties of the composition, e.g by being a hygroscopic and/or hyperosmotic ingredient and/or a bacterial desiccant.

According to an embodiment, the present invention concerns a composition, wherein at least one additional ingredient is present, which is a solvent.

According to an embodiment, the present invention concerns a composition, wherein said solvent is selected among glycerol, alcohol and water.

According to an embodiment, the present invention concerns a composition, wherein said solvent is glycerol.

It is speculated that honey and/or glycerol may aid in softening or dissolving ear wax if present.

According to an embodiment, the present invention concerns a composition comprising glycerol and optionally one or more additional ingredients for the treatment of earache.

It is speculated that glycerol may act as a hygroscopic and/or hyperosmotic ingredient, relieving the eardrum of pressure. It is further speculated that glycerol may act as a bacterial desiccant, impeding growth of bacteria.

According to an embodiment, the present invention concerns a composition, produced without the use of biofilm.

According to an embodiment, the present invention concerns a composition, free from microorganisms.

According to an embodiment, the present invention concerns a composition, free from bacteria.

According to an embodiment, the present invention concerns a composition, free from probiotic bacteria and/or organisms.

According to an embodiment, the present invention concerns a composition, comprising less than 18%, preferably less than 15%, more preferred less than 10%, preferably less than 5%, more preferred less than 3%, preferably less than 2%, more preferred less than 1% w/w water.

According to an embodiment, the present invention concerns a composition, which is substantially free from water.

According to an embodiment, the present invention concerns a composition, having a viscosity of 5 - 10000, preferably 10 - 5000, more preferred 25 - 1000, preferably 50 - 500, more preferred 75-400, preferably 100 - 300, more preferred 150 - 250, preferably about 200 cP (centipoise).

According to an embodiment, the present invention concerns a composition, comprising 50 - 100%, preferably 55 - 99%, more preferred 60 - 98%, preferably 65 - 97%, more preferred 70 - 96%, preferably 75 - 95%, more preferred 80 - 90% w/w glycerol.

According to an embodiment, the present invention concerns a composition, comprising 1 - 50%, preferably 2 - 45%, more preferred 3 - 40%, preferably 4 - 35%, more preferred 5 - 30%, preferably 10 - 25%, more preferred 15 - 20% w/w honey.

According to an embodiment, the present invention concerns a composition, further comprising extract of Trigonella.

According to an embodiment, the present invention concerns a composition, further comprising at least one tannin.

According to an embodiment, the present invention concerns a composition comprising at least one tannin, preferably a flavonoid, and optionally at least one additional ingredient for the treatment of earache.

The tannins, abundant proteins in the plant kingdom, are known to be good at binding with many proteins. The procyanidin (PCD) fraction of the tannins contains big phenolic compounds with multiple structure units that may have selective protein binding properties.

Some of the PCDs isolated from Vitis vinifera and Sambucus nigra extracts have been shown to be capable of inhibiting virus growth. When these PCDs are introduced, e.g. in a hypertonic, viscous solution, an osmotically active film is formed over a surface, attracting hypotonic liquid from the surface and detaching the bacteria by mechanical effect. By addition of honey, viscosity and anti microbial activity is improved. This presents topical virus neutralizing, protease inhibitor and antibacterial activities simultaneously.

Flavonoids comprise the subclasses flavonols, flavones, flavanones, flavan-3-ols and anthocyanidins.

Proanthocyanidins (PAs), the polymers of flavan-3-ols, also referred to as 'Condensed Tannins', are known for contributing astringent flavor to foods. It has been hypothesized that the free radical scavenging properties of PAs may reduce the risk of cardiovascular diseases, cancer, blood clotting and certain types of trimeric PAs may protect against some infections.

Procyanidins are members of the proanthocyanidin class of flavonoids. They are oligomeric compounds, and may be formed from catechin and epicatechin molecules.

According to an embodiment, the present invention concerns a composition further comprising at least one flavonoid.

According to an embodiment, the present invention concerns a composition, wherein said at least one flavonoid is a dietary flavonoid.

According to an embodiment, the present invention concerns a composition comprising at least one flavonoid selected among the group consisting of at least one flavonol, at least one flavone, at least one flavanone, at least one flavan-3-ol, and at least one anthocyanidin.

According to an embodiment, the present invention concerns a composition wherein at least one flavonoid is a proanthocyanidin.

According to an embodiment, the present invention concerns a composition according to any of the preceding claims, comprising at least one proanthocyanidin selected among the group consisting of at least one procyanidin, at least one prodelphinidin, and at least one propelargonidin.

According to an embodiment, the present invention concerns a composition comprising at least one procyanidin.

According to an embodiment, the present invention concerns a composition comprising multiple procyanidins.

According to an embodiment, the present invention concerns a composition comprising at least one procyanidin, wherein said at least one procyanidin is selected among an A-type procyanidin and a B-type procyanidin.

According to an embodiment, the present invention concerns a composition for the treatment of otitis.

According to an embodiment, the present invention concerns a composition for the treatment of otitis externa or otitis media.

According to an embodiment, the present invention concerns a composition for topical application.

According to an embodiment, the present invention concerns a composition for application in the outer part of the ear.

According to an embodiment, the present invention concerns a composition for application in the ear canal and/or on the surface of the eardrum.

According to an embodiment, the present invention concerns a composition for application with a cotton stick or a sponge.

According to an embodiment, the present invention concerns a composition for spray application.

According to an embodiment, the present invention concerns a composition for application via a tube or a syringe.

According to an embodiment, the present invention concerns a composition which is selected among the group consisting of lotion, shake lotion, cream, ointment, gel, foam, powder, paste, ear drop, and tincture.

According to an embodiment, the present invention concerns a composition comprising at least one ingredient selected among Vitis vinifera extract, Sambucus nigra extract, and Camellia sinensis extract.

According to an embodiment, the present invention concerns a spray comprising a composition of the invention.

According to an embodiment, the present invention concerns a method for treatment of earache, comprising topical application of a composition according to the invention.

According to an embodiment, the present invention concerns a method for treatment of earache, comprising increasing the osmotic pressure inside the ear channel by topical application of a composition comprising a pharmaceutically acceptable hyperosmotic ingredient. This allows establishing an osmotic pressure difference, allowing liquid to be transported away, alleviating pressure at the affected site.

According to an embodiment, the present invention concerns a use of a hyperosmotic ingredient for the treatment of earache.

According to an embodiment, the present invention concerns a use of honey and/or glycerol for the treatment of earache.

All cited references are incorporated by reference.

The accompanying Examples are provided to explain rather than limit the present invention. It will be clear to the person skilled in the art that aspects, embodiments, items and claims of the present invention may be combined.

### Examples

Unless otherwise mentioned, all ratios are w/w.

A composition according to the invention may for example be manufactured by mixing honey, glycerol and a procyanidin.

A composition according to Example 4 below was manufactured by mixing the ingredients. This composition provided very fast relief after topical application in the ear canal for a patient suffering from earache.

### Example 1

Liquid extract of Vitis vinifera seed (25.0% tannins): 5.4 g
Glycerol: QSP 100 g

### Example 2

Dry extract of Vitis vinifera seeds: 0.36 g
Honey: 19.0 g
Glycerol: QSP 100 g

### Example 3

Purified dried tannins from Sambucus nigra fruit: 0.2 g
Honey: 19.0 g
Glycerol: QSP 100 g

### Example 4

Dry extract of Vitis vinifera seeds: 1.5 g
Dry extract of Simbucus nigra fruits: 0.5 g
Honey: 19.0 g
Glycerol: QSP 100 g

### Example 5: Powder formulation

Dry powder of Vitis vinifera seeds: 10.0 g
Talcum powder: QSP 100 g

### Example 6

Dried fruit skin extract of Vitis vinifera: 0.74 g
Dried fruit extract of Simbucus nigra: 0.12 g
Honey: 5.0 g
Water: QSP 100 g

### Example 7

Dried extract of Vitis vinifera: 0.6 g
Dried extract of Simbucus nigra: 0.2 g
Honey: 20.0 g
Glycerol: 75.0 g
Water: QSP 100 g

### Items

The following items represent possible aspects and embodiments of the present invention.
1. Composition for the treatment of earache, said composition comprising a pharmaceutically acceptable hyperosmotic ingredient.
2. Composition according to item 1, wherein said hyperosmotic ingredient is selected among honey and glycerol.
3. Composition comprising:
   a. Honey and
   b. Optionally at least one additional ingredient for the treatment of earache.
4. Composition according to item 3, wherein:
   b. At least one additional ingredient is present, which is a solvent.
5. Composition according to item 4, wherein:
   b. Said solvent is selected among glycerol, alcohol and water.
6. Composition according to item 4, wherein:
   b. Said solvent is glycerol.
7. Composition according to any of the preceding items, produced without the use of biofilm.
8. Composition according to any of the preceding items, free from microorganisms.
9. Composition according to any of the preceding items, free from bacteria.
10. Composition according to any of the preceding items, free from probiotic bacteria and/or organisms.
11. Composition according to any of the preceding items, comprising less than 18%, preferably less than 15%, more preferred less than 10%, preferably less than 5%, more preferred less than 3%, preferably less than 2%, more preferred less than 1% w/w water.
12. Composition according to any of the preceding items, which is substantially free from water.
13. Composition according to any of the preceding items, having a viscosity of 5 - 10000, preferably 10 - 5000, more preferred 25 -1000, preferably 50 - 500, more preferred 75 - 400, preferably 100- 300, more preferred 150- 250, preferably about 200 cP (centipoise).
14. Composition according to any of the preceding items, comprising 50 - 100%, preferably 55 - 99%, more preferred 60 - 98%, preferably 65 - 97%, more preferred 70 - 96%, preferably 75 - 95%, more preferred 80 - 90% w/w glycerol.
15. Composition according to any of the preceding items, comprising 1- 50%, preferably 2 - 45%, more preferred 3 - 40%, preferably 4 - 35%, more preferred 5 - 30%, preferably 10 - 25%, more preferred 15 - 20% w/w honey.
16. Composition according to any of the preceding items, further comprising extract of Trigonella.
17. Composition according to any of the preceding items, further comprising:
   c. At least one tannin.
18. Composition according to any of the preceding items, further comprising:
   c. At least one flavonoid.
19. Composition according to item 18, wherein:
   c. Said at least one flavonoid is a dietary flavonoid.
20. Composition according to any of the preceding items, comprising:
   c. At least one flavonoid selected among the group consisting of at least one flavonol, at least one flavone, at least one flavanone, at least one flavan-3-ol, and at least one anthocyanidin.
21. Composition according to item 18, wherein:
   c. At least one flavonoid is a proanthocyanidin.
22. Composition according to any of the preceding items, comprising:
   c. At least one proanthocyanidin selected among the group consisting of at least one procyanidin, at least one prodelphinidin, and at least one propelargonidin.
23. Composition according to any of the preceding items, comprising:
   c. At least one procyanidin.
24. Composition according to any of the preceding items, comprising:
   c. Multiple procyanidins.
25. Composition according to any of the preceding items, comprising:
   c. At least one procyanidin, wherein said at least one procyanidin is selected among an A-type procyanidin and a B-type procyanidin.
26. Composition according to any of the preceding items, for the treatment of otitis.
27. Composition according to any of the preceding items, for the treatment of otitis externa or otitis media.
28. Composition according to any of the preceding items, for external application.
29. Composition according to any of the preceding items, for topical application.
30. Composition according to any of the preceding items, for application in the outer part of the ear.
31. Composition according to any of the preceding items, for application in the ear canal and/or on the surface of eardrum.
32. Composition according to any of the preceding items, for application with a cotton stick or a sponge.
33. Composition according to any of the preceding items, for spray application.
34. Composition according to any of the preceding items, for application via a tube or a syringe.
35. Composition according to any of the preceding items, which is selected among the group consisting of lotion, shake lotion, cream, ointment, gel, foam, powder, paste, ear drop, and tincture.
36. Composition according to any of the preceding items, comprising at least one ingredient selected among Vitis vinifera extract, Sambucus nigra extract, and Camellia sinensis extract.
37. Spray comprising a composition according to any of the preceding items.
38. Method for treatment of earache, comprising topical application of a composition according to any of the items 1 - 36.
39. Method for treatment of earache, comprising increasing the osmotic pressure inside the ear channel by topical application of a composition comprising a pharmaceutically acceptable hyperosmotic ingredient.
40. Use of a hyperosmotic ingredient for the treatment of earache.
41. Use of honey and/or glycerol for the treatment of earache.

## Claims

1. Composition for the treatment of earache, said composition comprising a pharmaceutically acceptable hyperosmotic ingredient.

2. Composition according to claim 1, wherein said hyperosmotic ingredient is selected among honey and glycerol.

3. Composition comprising:
a. Honey and
b. Optionally at least one additional ingredient
for the treatment of earache.

4. Composition according to claim 3, wherein:
c. At least one additional ingredient is present, which is a solvent.

5. Composition according to claim 4, wherein:
c. Said solvent is selected among glycerol, alcohol and water.

6. Composition according to claim 4, wherein:
c. Said solvent is glycerol.

7. Composition according to any of the preceding claims, produced without the use of biofilm.

8. Composition according to any of the preceding claims, free from microorganisms.

9. Composition according to any of the preceding claims, free from bacteria.

10. Composition according to any of the preceding claims, free from probiotic bacteria and/or organisms.

11. Composition according to any of the preceding claims, comprising less than 18%, preferably less than 15%, more preferred less than 10%, preferably less than 5%, more preferred less than 3%, preferably less than 2%, more preferred less than 1% w/w water.

12. Composition according to any of the preceding claims, which is substantially free from water.

13. Composition according to any of the preceding claims, having a viscosity of 5 - 10000, preferably 10 - 5000, more preferred 25 -1000, preferably 50 - 500, more preferred 75 - 400, preferably 100 - 300, more preferred 150 - 250, preferably about 200 cP (centipoise).

14. Composition according to any of the preceding claims, comprising 50 - 100%, preferably 55 - 99%, more preferred 60 - 98%, preferably 65 - 97%, more preferred 70 - 96%, preferably 75 - 95%, more preferred 80 - 90% w/w glycerol.

15. Composition according to any of the preceding claims, comprising 1- 50%, preferably 2 - 45%, more preferred 3 - 40%, preferably 4 - 35%, more preferred 5 - 30%, preferably 10 - 25%, more preferred 15 - 20% w/w honey.
